(19) Europäisches Patentamt
European Patent Office
Office européen des brevets

(11) Publication number: 0 082 146
B1

(12) EUROPEAN PATENT SPECIFICATION

(45) Date of publication of patent specification: 02.09.87

(51) Int. Cl.[4]: A 61 K 33/00, A 61 K 35/14, A 61 K 49/04, A 61 M 5/00, B 01 D 17/00, G 01 N 31/00, G 01 N 31/22, G 01 N 33/48, G 01 N 33/52

(21) Application number: 81902106.4

(22) Date of filing: 29.06.81

(86) International application number: PCT/US81/00898

(87) International publication number: WO 83/00088 20.01.83 Gazette 83/02

# (54) CHEMOTHERAPEUTIC AGENT AND TRACER COMPOSITION AND SYSTEM FOR USE THEREOF.

(43) Date of publication of application:
29.06.83 Bulletin 83/26

(45) Publication of the grant of the patent:
02.09.87 Bulletin 87/36

(84) Designated Contracting States:
DE FR GB SE

(56) References cited:
US-A-3 459 176
US-A-3 785 772
US-A-3 814 939
US-A-3 833 724
US-A-4 123 353
US-A-4 216 770
US-A-4 329 986

A.L. Babb, "Carbamylated Red Cell Distributions in Blood Treated Extracorporeally With Cyanate", publ. July 1977, by University of Washington, (Seattle, WA, USA), A Final Report prepared for: The Division of Blood and Diseases Resources, National Heart, Lung and Blood Institute Bethesda, MD.

(73) Proprietor: BIOMEDICS, INC.
3158 River Road
Des Plaines, IL 60018 (US)

(72) Inventor: BABB, Albert L.
3237 Lakewood Avenue South
Seattle, WA 98144 (US)
Inventor: BRUMFIELD, Robert C.
455 S. Oakland Avenue
Pasadena, CA 91101 (US)

(74) Representative: Heath, Derek James
Bromhead & Co., 30 Cursitor Street
Chancery Lane
London EC4A 1LT (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 082 146 B1

Courier Press, Leamington Spa, England.

**Description**

This invention relates to compositions and systems suitable for the extracorporeal treatment of whole blood.

Sickle cell anemia, the world's most common molecular disease, is the result of a single amino acid substitution at a surface position on the beta chain of hemoglobin. The only difference in primary chemical structure between normal hemoglobin (HbA) and sickle cell hemoglobin (HbS) is the substitution of valine for glutamic acid at the sixth amino acid from the $NH_2$-terminal of the beta chain.

The treatment of sickle cell anemia by inhibition of sickling using techniques of protein engineering is known. In particular, it is known that cyanate is useful to prevent the sickling of red blood cells of sickle cell anemia patients. See, for example U.S. Patent 3,833,724 to Cerami et al. It is also known that isocyanic acid, the reactive form of cyanate will react irreversibly with free amino groups of hemoglobin, primarily at the terminal valine position to decrease the polymerization of deoxyhemoglobin S molecules.

In HbS, the resulting carbamylation of the amino termini in the α- and β-chains that are present produces definite functional alterations such as an increase in oxygen affinity and a reduction in the Bohr effect. It is believed that carbamylation of the amino termini removes some of the salt bridges that stabilize the deoxy configuration of the hemoglobin tetramer present. Thus, carbamylation of HbS amino termini provides species of molecules which may not participate in the formation of tactoids. It has also been demonstrated that carbamylation of HbS prolongs considerably the survival of the red cells of the HbS homozygocytes without considerably affecting the red cell metabolism.

There are two major problem areas related to the use of cyanate in patients, however. The first problem area involves toxic effects due to non-specific carbamylation. Although the findings from animal experimentation appear rather optimistic, it is known that cyanate is a very reactive chemical with no specific affinity for hemoglobin. Intravenous or intraperitoneal administration of cyanate in mice has been shown to produce carbamylation of several enzymes in tissues other than blood, including the brain. Similar effects have been observed in *Macacca Nemestrina* after chronic administration of cyanate. Although the functional significance of this non-specific carbamylation remains to be assessed, such findings point to the need for maximum care, particularly in the chronic intravenous use of this drug.

The second problem area involves achieving effective levels of hemoglobin modifications *in vivo*. From the available *in vitro* evidence, it apears that protection from sickling requires the carbamylation of at least one amino terminal valine per HbS tetramer. Such levels of carbamylation are not easily achieved with oral administration of non-toxic doses of cyanate; the reported carbamylation levels in homozygous sicklers treated with cyanate by mouth have been about 0.3 carbamyl groups per tetramer. This low degree of carbamylation may explain the relatively unimpressive effects in patients routinely treated with cyanate orally. An effective degree of carbamylation with intravenous administration of cyanate in an attempt to affect a sickling crisis is out of the question because toxic doses of the drug may be reached before one achieves the desired therapeutic effects. On the other hand, the intravenous administration of 5 to 10 gm of cyanate ($LD_{50}$ = 250 mg/kg) to sickle cell anemia patients, has resulted in only 0.4 to 0.6 carbamyl groups per tetramer.

To overcome the foregoing problems, extracorporeal treatment of whole blood with cyanate has been suggested, followed by removal of unreacted cyanate from the treated blood by hemodialysis before the blood is returned to the patient. This can be done in a hemoreactor. In this manner, efficient carbamylation of HbS can be effected within a relatively short time period. However, in view of the recognized toxicity of cyanate, it is necessary to have a rapid, effective means for detecting the cyanate concentration in blood after hemodialysis so that the efficacy of cyanate removal during the hemodialysis step can be monitored and also so that free cyanate in the blood returned to the patient does not exceed a physiologically tolerable level.

To this end, it has been proposed to introduce cyanate into the blood to be treated as potassium cyanate (KNCO) and to monitor the cyanate concentration in whole blood by a pair of $K^+$-sensitive electrodes that compare the concentration of $K^+$ in blood before and after the treatment, Kjellstrand et al., *Trans. Amer. Soc. Artif. Int. Organs,* vol. *XX*, 574—577 (1974). However, the foregoing approach is not satisfactory because the measurement of $K^+$ concentration in whole blood does not give a reliable indication of the cyanate concentration that may be present because relatively large stores of potassium are already present in the patient's body.

The use of a standard hemodialysis system has its own problems. Inasmuch as the system pressure on the blood side of the membrane is higher, ultrafiltration takes place. As a result, water also passes through the semipermeable membrane with attendant and sometimes undesirable decrease in the patient's body mass during hemodialysis.

Accordingly, there exists a pressing need for a safe and reliable means for determining the concentration of the cyanate content of whole blood before a practical treatment can be provided to patients suffering from sickle cell anemia. The present invention satisfies this need.

From one aspect, the invention is directed to a therapeutic composition suitable for extracorporeal treatment of whole blood and comprising a water-soluble chemotherapeutic agent and a water-soluble fluorescable tracer means, characterised in that the chemotherapeutic agent and the tracer means are dialyzable from whole blood at rates that are a function of one another, and the fluorescable tracer means is

an N-substituted benzamide represented by the formula

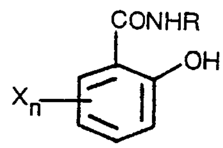

wherein R is hydrogen or methyl, X is a halogen, methoxy or ethoxy, and n is an integer having a value of 0 to 1, and physiologically-tolerable alkali metal salts thereof.

The invention also extends to a therapeutic composition suitable for extracorporeal treatment of whole blood and comprising an aqueous solution containing cyanate and salicylamide, characterised in that the cyanate is present in a concentration of 0.2 molar to 0.6 molar and the salicylamide is present in a concentration of 2.5 millimolar to 8.5 millimolar.

From yet another espect, the invention is directed to a method of treating whole blood with a dialyzable chemotherapeutic agent in an extracorporeal stream of the blood, characterised by:

(a) introducing the chemotherapeutic agent into the extracorporeal stream of blood together with a fluorescable tracer means that is dialyzable therefrom at a rate which is a function of the dialysis rate for the chemotherapeutic agent;

(b) dialyzing the extracorporeal stream so as to remove therefrom in a dialyzate the chemotherapeutic agent and the tracer means at respective rates that are a function of one another;

(c) irradiating a portion of the obtained dialyzate with fluorescence-exciting electromagnetic radiation;

(d) passing the irradiated portion over a detector means responsive to fluorescence emitted by the tracer means present in the said portion and generating an electrical signal indicative of the intensity of emitted fluorescence; and

(e) energizing an indicator means with the generated signal;

the fluorescable tracer means being an N-substituted benzamide represented by the formula:

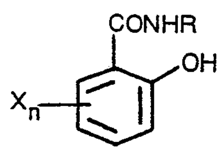

wherein R is hydrogen or methyl, X is a halogen, methoxy or ethoxy, and n is an integer having a value of 0 or 1, and physiologically-tolerable alkali metal salts thereof.

The invention still further extends to a method for determining the concentration of a dialyzable chemotherapeutic agent in an extracorporeal stream of whole blood containing the chemotherapeutic agent and salicylamide, characterised by:

(a) contacting at least a portion of the extracorporeal stream across a dialysis membrane with an aqueous alkaline solution devoid of salicylamide for a predetermined time period;

(b) thereafter recovering the aqueous alkaline solution;

(c) irradiating an aliquot of the recovered aqueous alkaline solution with ultraviolet radiation having a wavelength of about 318 nanometers;

(d) positioning the irradiated aliquot adjacent to a detector means generating an output signal in response to emitted radiation having a wavelength of about 410 nanometers, the signal being proportional to the intensity of the emitted radiation; and

(e) energizing an indicator means with the output signal.

The features of the present invention will become readily apparent from the following detailed description of the invention with reference to the accompanying drawings, in which:

FIGURE 1 is a schematic illustration of an extracorporeal blood treatment system which makes use of the compositions and method of the present invention;

FIGURE 2 is an enlarged perspective view of a peristaltic pump means suitable for the system shown in FIGURE 1;

FIGURE 3 is a perspective view of a hemoreactor which is partially cut away to illustrate the interior structure;

FIGURE 4 is a cross-sectional view of the tubing forming a part of the apparatus; and

FIGURE 5 is a diagrammatic view of a portion of the tubing showing a pair of spiral wound spacer filaments on the exterior of the tubing.

The fluorescable tracer compounds suitable for the purposes of the present invention are N-monosubstituted benzamides represented by the formula

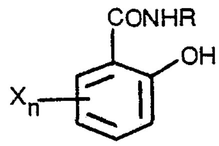

wherein R is hydrogen or methyl, X is halogen (fluorine, chlorine, bromine or iodine) methoxy or ethoxy, and n is an integer having a value of 0 or 1. Physiologically-tolerable alkali metal salts of the foregoing compounds, e.g., the sodium salts, are also suitable.

Salicylamide is a particularly preferred tracer compound. Salicylamide (M. W. about 137.12), also known as o-hydroxybenzamide, is commercially available and is frequently utilized as a constituent of pain remedies. Salicylamide has no known tendency to bind to blood proteins or to interfere with platelet aggregation. When introduced into a mammalian blood stream intracorporeally, it is present in the plasma fraction predominantly in the conjugated form. The plasma levels of salicylamide remain extremely low, presumably due to accumulation in tissues and rapid excretion via the kidneys.

Upon excitation by ultraviolet radiation having a wavelength of about 318 nanometers, salicylamide fluoresces with a blue light having a wavelength about 410 nanometers. The intensity of fluorescence is dependent on concentration, pH and temperature, thus for the monitoring of concentration changes in a given salicylamide-containing solution, the pH and temperature should be controlled. Preferably, the pH value of the salicylamide-containing solution is maintained in a range within $\pm0.05$ and the temperature is maintained within a range of about $\pm0.5$°C. To this end it is desirable to use a buffered solution the temperature of which is controlled within the desired limits. A suitable buffer for this purpose is the monobasic potassium phosphate buffer (pH=7.4). Such buffers are commercially available.

As used herein and in the appended claims, the term "cyanate" is intended to mean a compound providing or containing a reactive cyanate (—NCO) group. For carbamylation of HbS one such suitable compound is isocyanic acid (HNCO) either in its ionized or unionized form. A suitable source of isocyanic acid, in turn, can be sodium cyanate (NaNCO) which is in equilibrium with isocyanic acid when in an aqueous medium, i.e.:

$$NaNCO + H_2O \rightleftharpoons HNCO + Na^+ + OH^-$$

Other alkali metal cyanates, e.g., potassium cyanate, can also be used. In general, any water-soluble physiologically tolerable cyanate salt can be used. Also suitable as cyanate sources are the dialyzable alkyl ureas of which the $C_1$ to $C_4$ alkyl ureas, e.g., N,N'-dimethylurea, N,N-diethylurea, N-methyl-N'-butylurea, N,N'-dipropylurea, N-ethylurea and N-butylurea are preferred.

Other suitable chemotherapeutic agents that can be used in conjunction with a fluorescable tracer compound for the extracorporeal treatment of the blood of sickle cell anemia patients are the nitrogen mustards, the alkyl acetimidates, e.g., methyl acetimidate, the dialkyl adipimates, e.g., dimethyl adipimate.

In the case of carbamylation by a cyanate, the therapeutic compositions of the present invention can be compounded in dry form. A preferred therapeutic composition has a cyanate-to-salicylamide weight ratio of 20:1 to 50:1, respectively and preferably a respective weight ratio of about 35:1. Alternatively, the present compositions can be aqueous solutions containing the foregoing ingredients. The cyanate can be present in a concentration of 0.2 molar to 0.6 molar, and salicylamide can be present in a concentration of 2.5 millimolar to 8.5 millimolar. A cyanate-to-salicylamide molar ratio of about 75 is preferred.

If desired, buffering agents and anticoagulants such as heparin or trisodium citrate may also be present.

A typical composition embodying the present invention is compounded as follows:

| | |
|---|---|
| sodium cyanate | 27.30 grams |
| salicylamide | 0.77 grams |
| sterile water, q.s. to | 1000 milliliters |

The pH of the resulting aqueous solution is then adjusted to a desired alkalinity, usually to a pH of about 8 or higher, using a physiologically tolerable alkaline compound, e.g., NaOH or a buffer, the solution is filtered, e.g., through a 0.22-µm filter commercially available from Millipore Corporation, and thereafter packaging in conventional intravenous containers. The solution can also be prepared using an isotonic (pH 7.4) buffer solution such as Normosol-R[1], commercially available from Abbott Laboratories, North Chicago, Illinois.

For chemotherapy of a patent afflicted with sickle cell anemia, the foregoing aqueous solution is infused into an extracorporeal blood stream of the patient passing through a suitable hemoreactor, e.g., a cartridge comprising a coil or a plurality of hollow tubes, at a volumetric flow rate ratio of 30:1 to 10:1, that is, at a blood flow rate of 30 to 10 milliliters per minute the cyanate- and salicylamide-containing aqueous solution is infused at a rate of about one milliliter per minute. A preferred residence time of the blood within

[1] Per 100 milliliters of aqueous solution Normosol-R contains the following dissolved ingredients:

the hemoreactor is about ten minutes.

For carbamylation of HbS, the concentration of cyanate in the patient's extracorporeal blood stream can be in the range of 0.01 molar to 0.03 molar, and preferably is about 0.02 molar. Usually about 10 weight percent of the cyanate present reacts with HbS during the treatment. The salicylamide concentration in the extracorporeal blood stream during the foregoing carbamylation treatment can be 0.14 millimolar to 0.4 millimolar, and preferably is about 0.27 millimolar.

| | | | |
|---|---|---|---|
| NaCl | 526 mg | Na | 140 meq/l |
| Na acetate | 222 mg | K | 5 meq/l |
| Na gluconate | 502 mg | Mg | 3 meq/l |
| KCl | 37 mg | Cl | 98 meq/l |
| $MgCl_2$ | 14 mg | Acetate | 27 meq/l |
| | | Gluconate | 23 meq/l |

Thereafter ureacted cyanate together with salicylamide are removed from the treated blood stream in a hemodialyzer using a conventional dialysate. A typical dialysate suitable for this purpose has the following composition [1]:

| | |
|---|---|
| sodium ion | 135 meq/liter |
| chloride ion | 101 |
| magnesium ion | 1 |
| acetate ion | 38 |

After dialysis of the extracorporeal blood stream has been effected, the concentration of salicylamide, and thus cyanate, in this blood stream is ascertained by subjecting the dialysate to fluorometric analysis either directly or after a dilution, as desired for optimum determination of concentration. Alternatively, the blood stream, or a portion thereof, can be pased through a hemoanalyzer which in effect is a secondary dialyzer in which an aqueous alkaline solution, e.g., an isotonic saline solution or a Normosol-R solution having a predetermined alkaline pH, at a predetermined temperature, passes countercurrent to the blood stream as a secondary dialysate solution devoid of salicylamide. A conventional dialysis membrane, e.g., of the type shown in U.S. Patent No. 4,031,012 to Gics, or the like, separates the secondary dialysate solution from the blood stream. The residence time of the respective countercurrent streams within the streams within the hemoanalyzer preferably is selected so that equilibration of the salicylamide concentration on both streams across the dialysis membrane is substantially achieved. Thereafter the obtained dialysate is subjected to fluorometric analytical techniques so as to measure intensity of fluorescence and thus the salicylamide concentration therein, e.g., by using a commercially available fluorometer, such as a Turner Spectrofluorometer or the like. Fluorescable tracer means other than salicylamide can be handled in a similar manner.

During fluorometric analysis, the dialysate preferably is maintained at a pH and temperature that optimizes fluorescence. For salicylamide a pH of about 7.4 and a temperature of about 41°C. are preferred. However, the pH value can vary within the range of 7.35 to 7.45 and the temperature within the range of 39.5°C. to 41.5°C. In any event, during fluorometry the aqueous dialysate solution containing the fluorescable tracer compound, e.g., salicylamide, should be maintained as closely as possible at the same pH and temperature as was used for calibration of the fluorometer since fluorometry is quite sensitive to both pH and temperature.

The relative flow rates of the blood stream and the secondary dialysate in any given instance will depend, of course, on the type of hemo-analyzer used, the characteristics of the semipermeable membranes that separate the two streams, and similar factors. In a typical instance, a dialysate flow rate of about 0.2 milliliters per minute through the tube side of a shell-and-tube hemo-analyzer comprising hollow, semi-permeable fibers as the tubes and a blood flow rate of about 40 milliliters per minute or less through the shell side of the hemo-analyzer will permit a degree of equilibration of nearly 100 percent.

The present invention is further illustrated by the following example.

Example 1

Determinaton of Cyanate
Content of Blood Stream

Whole blood is contacted in a hemo-reactor with an aqueous solution of sodium cyanate (about 0.42 molar) and salicylamide (about 5.6 millimolar) at a flow rate sufficient to maintain a blood stream HNCO concentration of about 20 millimolar during the treatment and is then detoxified in a Travenol CF 1500 hollow fiber dialyzer, commercially available from Travenol Laboratories, Deerfield, Illinois, having an effective dialysis area of about 1.5 square meters. The dialysate composition is as follows:

---

[1] For use with trisodium citrate as anticoagulant; since the dialysate contains no calcium, $Ca^{++}$ is subsequently added as heparinized aqueous $CaCl_2$ solution before the treated and dialyzed blood is returned to the patient. If heparin is the only anticoagulant used, then calcium ion can be present in the dialysate.

| | |
|---|---|
| sodium ion | 135 meq/liter |
| chloride ion | 101 meq/liter |
| magnesium ion | 1 meq/liter |
| acetate ion | 38 meq/liter |

The blood flow rate through the dialyzer is about 40 milliliters/minute.

The concentration of salicylamide in the blood stream leaving the dialyzer is determined by subjecting this blood stream to a secondary dialysis with Normosol-R at 35°C. and pH 7.4. The obtained secondary dialysate is then subjected to fluorometric analysis by irradiating with UV radiation having a wavelength of about 318 nanometers and measuring the intensity of fluorescence at about 410 nanometers using a photomultiplier tube (RCA 4552) and optical filters (Schott KV—418, BG—12 and UG—1). Utilizing the known ratio of cyanate to salicylamide in the infusate, the dialysis rates for cyanate and salicylamide, and a calibration curve of fluorescence intensity versus salicylamide concentration, the following information is obtained:

| Cyanate Removal Efficienty in Dialyzer percent | Fluorescence Intensity, Millivolts | Cyanate Concentration, Millimolar |
|---|---|---|
| 99 | 100 | 0.21 ± 0.01 |
| 98 | 122 | 0.40 ± 0.02 |
| 97 | 167 | 0.60 ± 0.03 |
| 96 | 239 | 1.0 ± 0.05 |
| 95 | 389 | 2.0 ± 0.10 |

While in the foregoing example salicylamide is the fluorescent tracer compound that is utilized in conjunction with a cyanate as the dialyzable, water-soluble therapeutic agent, other fluorescable tracer compounds of the above general formula that do not bind to plasma proteins can be used as well. The fluorescable tracer compounds of the above general formula, of course, have to be water-soluble, and dialyzable, and the extraction efficiency during dialysis preferably should approximate that of the chemotherapeutic agent. Preferably the water solubility of the fluorescable tracer compound of the above general formula at 37°C. is at least about 1.0 grams/liter. For use in the treatment of sickle cell anemia, by carbamylation, the fluorescable tracer compound of the above general formula preferably should fluoresce at about 40°C. ± 0.1° and at a pH of about 7.4 ± 0.05.

While in some cases a portion of the dialysate can be analyzed fluorometrically in view of the relatively high concentration of salicylamide or similar fluorescable tracer compound of the above general formula in the dialysate that may be present, usually it is desirable to dilute the dialysate portion that is to be subjected to fluorometric analysis in order to enhance accuracy of the measurement. From the flow rates of the respective fluids introducing the tracer compound into the system and removing the tracer compound from the system, and from the degree of dialysate dilution, if any, during fluorometric analysis the residual amount of the tracer compound, if any, that remains in the blood stream returned to the patient after extracorporeal treatment can be calculated using conventional material balance procedures. An appropriately programmed microprocessor is particularly well suited for this purpose. Once the concentration of the tracer compound in the blood stream being returned to the patient is determined, the maximum possible concentration of cyanate in that blood stream is ascertained. Inasmuch as at least some of the cyanate initially introduced into the blood stream will have reacted with HbS, the actual cyanate concentration in the return blood stream will, of course, be less than the ascertained maximum and the fluorometric determination of concentration will be conservative.

Alternatively, the concentration of the chemotherapeutic agent remaining in the blood stream after hemodialysis can be ascertained by removing the tracer compound remaining in the dialyzed whole blood, if any is present, during a secondary dialysis step in the form of an aqueous dialysate or extract. The concentration of the tracer compound in this extract also is a known function of the concentration of the tracer means in the dialyzed whole blood and thus also proportional to the residual concentration of the chemotherapeutic agent in the dialyzed whole blood. Preferably, the secondary dialysate is substantially equilibrated with the dialyzed whole blood so that the concentration of the tracer compound, such as salicylamide, in the secondary dialysate is equal to or closely approximates the concentration of the tracer compound in the blood stream being returned to the patient.

In either case a dialysate portion is then irradiated with fluorescence-exciting electromagnetic radiation, and the intensity of the emitted fluoroescence is ascertained by generating, by means of an appropriate detector such as a photomultiplier tube and associated circuitry, an electrical signal having a magnitude that is indicative of the intensity of the emitted fluorescence. The electrical signal, in turn, is used to energize an indicator means such as a meter, a warning light or buzzer, a recorder pen, or the like.

The apparatus to carry out this treatment of blood is shown in FIGURES 1 to 5. FIGURE 1 illustrates a continuous, substantially constant volume system for the extracorporeal treatment of a body fluid such as blood. The system provides a continuous, confined flow blood passageway defined by blood treatment

and analysis vessel and conduits to and from such vessels. The confined flow blood passageway, in turn, defines an extracorporeal loop or circuit for circulating a portion of the patient's blood. During blood treatment the extracorporeal loop is, of course, connected to the patient's cardiovascular system.

Such connection can be conveniently made by first surgically establishing a channel between a neighboring artery and a vein so as to convert and enlarge adjacent venous blood vessels into high pressure and high flow rate vessels. A channel surgically established in the foregoing manner is known as an "A—V fistula". A common location for an A—V fistula is the patient's forearm.

Vascular access for the withdrawal of the requisite volumes of blood for extracorporeal treatment is established by penetrating the patient's blood vessels with a hypodermic needle through the patient's skin. The hypodermic needle in turn is connected to flexible tubing 11 which is a part of the extracorporeal loop of the present system and serves as a conduit for the blood withdrawn from a patient.

In addition to flexible tubing 11, the extracorporeal loop is defined by tee 28, flexible tubing 12 having a portion that passes through blood pump 19, tee 21, tubing 13, drip chamber 22, hemoreactor 23, conduit 14, hemodialyzer 24, conduit 15, tee 25, conduit 16, hemoanalyzer vessel 26, conduit 17, tee 35, drip chamber 27, and flexible tubing 18 having a portion that passes through blood pump 19 and which serves as the conduit for returning treated blood to the patient via another connection usually effected by means of a hypodermic needle. Blood pump 19 is driven by prime mover 20 such as an electric motor or the like.

The particular extracorporeal loop illustrated in FIGURE 1 is intended for carbamylation of HbS in the blood stream of a sickle cell anemia patient; however, the nature and type of blood treatment vessels within the extracorporeal loop can vary depending on the desired blood treatment. The present system for extracorporeal treatment of blood is not limited to HbS carbamylation but is equally well suited for hemodialysis as well as for other treatments that utilize a patient's blood stream as vehicle for chemotherapeutic agents.

Blood pump 19 is a dual action, variable speed peristaltic pump for withdrawing blood from the patient via flexible tubing 11 and 12, and for returning treated blood to the patient via flexible tubing 18. Those portions of flexible tubing 12 and 18 within blood pump 19 are of substantially uniform inside diameter, thus per unit time blood pump 19 introduces a constant volume of blood into the extracorporeal loop and withdraws substantially the same volume from this loop for return to the patient. In this manner, ultrafiltration in hemodialyzer 24 is substantially reduced and occurs only to the extent of extraneous infusate volume introduced into the extracorporeal loop during a specific blood treatment. Additionally, the foregoing arrangement permits the monitoring of the total liquid volume that is being returned to the patient from the extracorporeal loop.

The various tees forming a part of the confined flow blood passageway are provided so as to permit the introduction of anticoagulants and/or other blood additives, chemotherapeutic agents, or specific substances or compounds for the calibration of analytical or monitoring instruments that may be present in the extracorporeal blood circuit. In particular, tee 28 is provided for connecting anticoagulant source 29 into the loop via conduit 30 and anticoagulant pump 31. Similarly, tee 21 downstream from blood pump 19 is provided for connecting chemotherapeutic agent source 32 into the loop via conduit 33 and drug pump 34, and tee 35, downstream from hemoanalyzer 26 but upstream from drip chamber 27, is provided for connecting optional blood calcium supplement source 36 into the loop via conduit 37 and calcium pump 38. The optional blood calcium supplement source 36 can be connected into the system for use in instances where the anticoagulant introduced into the extracorporeal blood circuit is trisodium citrate. In instances where the anticoagulant used in heparin, calcium ions can be present in the dialysate and need not be separately introduced. Also, the location of drip chamber 27 in the extracorporeal blood circuit as shown in FIGURE 1 is illustrative but not critical. For example, in instances where appropiate transducers are operably associated with drip chamber 27 to monitor the patient's venous blood pressure, drip chamber 27 should be positioned downstream from blood pump 19 so that the observed venous blood pressure readings are not influenced by the pumping action provided by blood pump 19.

Preferably conduits 30, 33 and 37 are made of flexible tubing. Auxiliary pumps 31, 34, and 38 preferably are peristaltic metering pumps that can coact with the flexible tubing to dispense the requisite amounts of additives with accuracy and are driven in synchronism with blood pump 19. More preferably, the energization circuits of pumps 31, 34 and 38 are interconnected with the energization circuit for blood pump 19 so that when blood pump 19 is de-energized these auxiliary pumps stop as well.

As a precautionary measure, bubble detector 39 and associated line clamp 40 are provided in blood return conduit 18 downstream from blood pump 19.

Initial treatment of the blood withdrawn from the patient can be effected in hemoreactor 23 downstream from blood pump 19, tee 21 and drip chamber 22 by subjecting the blood passing therethrough to a desired temperature for a predetermined time period in the presence of a chemotherapeutic agent infused via conduit 33. The desired temperature conditions within hemoreactor 23 can be maintained by using as the heat transfer medium hot dialysate which thereafter is utilized to detoxify the treated blood in dialyzer 24 however, any other suitable heat transfer medium can be employed. The hot dialysate can be first introduced into hemoreactor 23 by way of conduit 41 and then transferred to dialyzer 24 by way of conduit 42. Spent dialysate exits from dialyzer 24 via conduit 43. A preferred configuration of the hemoreactor is discussed below.

On the other hand, in the case of blood treatment by hemodialysis alone, hemoreactor 23 can be

dispensed with, and blood from drip chamber 21 can be introduced directly into dialyzer 24.

In instances where a chemotherapeutic agent is introduced into the extracorporeal loop and subsequently removed as illustrated hereinabove, it is often desirable to monitor the residual concentration of the chemotherapeutic agent in the treated blood leaving the dialyzer. For this purpose, hemoanalyzer vessel 26 can be provided in the extracorporeal loop downstream from dialyzer 24 but upstream from blood pump 19. Hemoanalyzer vessel 26 shown in FIGURE 1 for purposes of illustration can be a secondary dialysis unit that utilizes an independent secondary dialysate from secondary dialysate source 44 which secondary dialysate is conveyed to hemoanalyzer vessel 26 via conduit 45 by means of metering pump 46. Dialysate emanating from vessel 26 is conveyed by way of conduit 47 through analyzer 48 and thereafter to drain 49. In use with a fluorescable tracer, the analyzer 48 includes a fluorescence-exciting electromagnetic radiation source and a photomultiplier as disclosed above. An electrical output signal generated by analyzer 48 can be fed to appropriate microprocessor, controller and/or alarm circuits by means of lead 50. If desired, drop counter 66 or similar flow monitoring means can be provided downstream from analyzer 48 to provide an indication that the secondary dialysate is flowing at the desired rate.

Blood pump 19 is the main peristaltic pump means for transporting blood into and out of the extracorporeal loop. An illustrative structural arrangement for blood pump 19 is shown in FIGURE 2.

Blood pump 19 is provided with arcuate housing 55 having bearing surface 65, open at one end, and accommodating segments of flexible tubing 12 and flexible tubing 18. Pump 19 is further provided with a pair of rollers such as roller 56 and roller 57, both rotatably mounted on respective carrier arms 58 and 59 and coacting with the adjacent segments of flexible tubing 12 and 18. Each of the carrier arms 58 and 59 is pivotally mounted on hub member 60 rotatably driven by means of shaft 61. Bias compression springs 62 and 63, mounted between hub member 60 and carrier arms 58 and 59, respectively, urge at least one of the rollers 56 and 57 at a time against portions of flexible tubing 12 and 18 held within arcuate housing 55 so as to substantially simultaneously occlude the tubing passageways. Rolling motion of rollers 56 and 57 along portions of flexible tubing 12 and 18 retained within arcuate housing 55 simultaneously transports substantially the same volume of blood into and out of the extracorporeal loop.

In this manner, each of rollers 56 and 57 coacts, one at a time in turn, with both flexible tubing 12 and flexible tubing 18 to form dual, simultaneously acting pumping elements. In the embodiment shown in FIGURE 2 both of these pumping elements are driven from common shaft 61 and further coact with arcuate housing 55 to occlude flexible tubing 12 and flexible tubing 18 and thus that segment of the extracorporeal loop within which the extracorporeal blood treatment device or devices are situated. At the same time, the rolling motion of rollers 56 or 57, whichever roller contacts tubing 12 and 18 at any given time, moves the produced occlusion in each tubing along bearing surface 65 of arcuate housing 55. As a result, movement of the occlusions produces pumping action that transports the patient's blood through the extracorporeal blood loop. In particular, the individual pumping action of the pumping elements formed by a roller and an occluded tubing portion, withdraws blood from the patient, circulates the withdrawn blood through the blood treatment device or devices, and returns the treated blood to the patient at substantially the same volumetric rate. Inasmuch as both pumping elements can be driven from the same shaft, the blood circulation rate in the extracorporeal loop can be readily adjusted by operating a single control, namely pump speed control 64. Also, in such a case the volume of liquid being returned to the patient from the extracorporeal loop is a function of the number of revolutions by shaft 61 and can be readily ascertained by monitoring the shaft revolutions per unit time, for example, by using a tachometer.

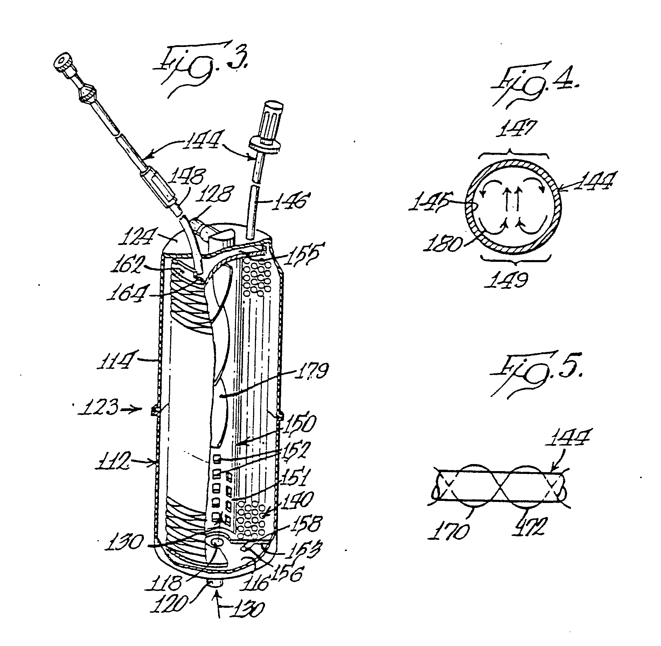

A hemoreactor which can be used in the system of FIGURE 1 is shown in FIGURES 3—5. The different positions of the conduits are to illustrate another embodiment and do not effect performance.

For optimum performance of the hemoreactor the following design criteria are significant to provide substantially plug flow of fluid stream to be treated, e.g., blood in admixture with a chemotherapeutic agent. The flow dispersion in this fluid stream preferably should be about 0.02 or lower, more preferably about 0.01. Flow dispersion in a liquid stream is given by the expression

$$\text{flow dispersion} = Du/L$$

where D is solute diffusivity in $cm^2$/minute, u is linear flow velocity in cm/minute, and L is length of flow path in cm.

The reactor of the present invention minimizes flow dispersion by providing a coiled conduit for the fluid stream to be treated, i.e., the first fluid stream, and thus inducing a substantially toroidal secondary flow therewithin. Intensity of the induced secondary flow is proportional to the square root of the Dean Number which can be calculated from the expression

$$\text{Dean No.} = Re\ (r_t/R_c)^{1/2}$$

where Re is Reynolds Number, $r_t$ is the inside radius of the conduit, and $R_c$ is the inside radius of the conduit coil. Thus, the greater the Dean Number the greater is the intensity of the induced secondary flow, and the closer the fluid flow within the conduit approximates plug flow. For a reactor embodying the present

invention the ratio of $r_t$ to $R_c$ preferably is about 0.025 to about 0.1, and more preferably about 0.04 to about 0.08.

The pressure drop across the conduit is also a significant factor and preferably should not exceed about 100 mm of mercury at the operational fluid flow rates with the Reynolds Number in the laminar flow region, and preferably at a value of less than about 200. In the case of blood chemotherapy, the fluid shear rate at the conduit wall should be greater that about 50 $sec^{-1}$ in order to avoid, or at least minimize, the Rouleaux effect. More preferably, for blood chemotherapy the fluid shear rate at the conduit wall should be greater than about 75 $sec^{-1}$.

Heat transfer efficiency is also important, particularly when the fluid to be treated is blood. For the latter, it is desirable that the average temperature within the blood-carrying conduit be maintained at a level as high as possible commensurate with limitations imposed by hemolysis and red blood cell survival rates in order to minimize necessary residence time within the reactor and thus the total time of treatment needed. To this end, it has been found that the blood temperature can be maintained as high as about 43°C. without adversely affecting the blood hematologically, and the effective heat transfer area of the conduit, as well as the flow rate of the heat transfer medium, i.e., the second fluid stream, through the reactor should be selected accordingly.

Referring now to FIGURE 3, the elements of the hemoreactor 123 embodying the present invention are illustrated. Hemoreactor 123 includes a casing or tank 112 having a generally cylindrical shell 114, which may be formed as two longitudinally aligned halves bonded together. If the hemoreactor is to be of the reusable type, shell 114 can be made of stainless steel or similar material. For disposable hemoreactors, on the other hand, shell 114 can be made of thermoplastic or thermosetting polymeric materials that are generally acceptable for use in medical equipment. The tank 112 includes a generally circular first end wall 116 defining an inlet orifice 118 for a heat transfer medium, i.e., a heating fluid or cooling fluid. Fluid inlet nozzle 120 projects outwardly from end wall 116. The tank 112 also includes a generally cylindrical second end wall 124 defining an outlet orifice (not visible in FIGURE 3) which includes an integral outlet nozzle structure 128. In FIGURE 3, the heating or cooling fluid entering through the inlet nozzle 120 flows upwardly through the tank 112 as indicated by arrows 30 and eventually exits through the discharge nozzle 128.

The fluid to be treated (such as blood and one or more of various therapeutic agents), flows in a convoluted conduit assembly 140 mounted within the tank 112 in a spaced relationship from the sidewalls of tank 112. In operation, the tank 112 is filled with the flowing heating or cooling fluid so that the assembly 140 is entirely submerged therewithin.

The conduit assembly 140 includes a convoluted conduit 114 having an inlet portion 146 and an outlet portion 148 passing through the end wall 124. The end wall 124 is sealed around the inlet and outlet portions 146 and 148 of conduit 144 to prevent out leakage of the heating or cooling fluid. Although the inlet and outlet portions 146 and 148 of conduit 144 are illustrated as passing through the same end wall 124 of the tank, it is to be realized that both inlet and outlet portions 148 could be arranged to pass through the other end wall 116 of the tank. Also, just one of the tubing portions, 146 or 148, could be arranged to pass through the end wall 116 with the remaining portion passing through end wall 124.

The conduit 144 may be made from any suitable material, such as polyvinyl chloride tubing typically having an OD of about 0.281 inch and an ID of about 0.165 to about 0.188 inch. In the case of blood treatment, the material, of course, should be non-thrombogenic. Inside of the tank 112, the tubing 144 is arranged in a plurality of concentric helices about a longitudinal axis which, in the preferred embodiment illustrated, is coincident with the longitudinal axis of the generally cylindrical tank shell 114. The resulting coiled structure is one in which a plurality of substantially co-axial helices are formed around the longitudinal axis of the tank 112 in layers of increasingly larger diameter.

In a preferred embodiment, the conduit 144 is wound around, and at least in part supported by, a spool member 150 comprising a generally cylindrical foraminous core 151 and core end flanges 153 and 155. In yet another preferred embodiment, the core can be solid, defining an annular space within which the convoluted conduit is situated and through which annular space the heat transfer medium can be circulated. If a solid core is utilized, core end flanges are, of course, omitted and the incoming heat transfer medium is distributed over the convoluted conduit by means of an appropriate header. The core may be made of any suitable material, such as polypropylene, and may be solid or foraminous, e.g., provided with a plurality of perforations 152 through which the heating or cooling fluid can be distributed.

As shown in FIGURE 3, foraminous core 151 is provided with end flanges 153 and 155 substantially parallel to one another and oriented in planes parallel to the plane defined by the end wall 116. To prevent leakage of the heating or cooling fluid between the flange 153 and the interior surface of the end wall 116, an O-ring 156 is positioned in a suitable receiving groove 158 provided in the flange 153. Preferably, the core 151 has an internal diameter equal to or greater than the tank inlet 118 defined within tank end wall 116 on the interior of the core 151 so that the heating or cooling fluid flows from the interior of the core radially outwardly through the core. The inlet and outlet portions of tubing 144 preferably pass through the core end flange 155 which, in turn, may be provided with suitable bores or apertures for receiving the tubing.

Core end flange 155 also functions as a baffle plate deining first and second generally cylindrical, axially aligned, internal chambers within the tank 112. The first chamber contains the helical configurations of conduit 144 and the second chamber is located adjacent the outlet end of tank 112 in communication with the discharge nozzle 128.

The core end flange 155 terminates radially short of the cylindrical shell 114 of the tank 112 to provide communication around the baffle plate between the first and second chambers so that a portion of the heating or cooling fluid flowing into the tank through the inlet orifice 118 can eventually flow around the core end flange 155 and out the tank discharge nozzle 128. The core end flange 155 and foraminous core 151 are retained in their proper axial positions within the tank 112 by means of a spacer ring 162 which is positioned between the inside surface of tank end wall 124 and the core end flange 155 and which may be secured to, or formed integrally with, the core end flange 155. The spacer ring 162 has appropriate cut outs or notches 164 to permit passage of the heating or cooling fluid around the core end flange 155.

By forming the conduit 144 into the plurality of helices illustrated in FIGURE 3, improved mixing and radial mass transport of non-turbulent flow conditions is obtained. Specifically, in FIGURE 4 secondary flow patterns within the cross section of the conduit 144 are illustrated. The conduit 144 is seen to have a generally circular internal flow passageway 145. The outer or convex region of the helix segment is designated by 149. Owing to the centrifugally-induced forces acting on the gross or primary fluid flow in the conduit, a radial mass transport in the form of a double toroidal secondary flow is thus produced generally transverse to the gross fluid flow in a pattern generally illustrated by the arrows 180. This secondary flow, imposed upon the primary flow, aids in heat transfer and mixing of the fluid constituents between the center of the passageway 145 and the periphery of the passageway 145 within the conduit 144.

Though not illustrated, additional mixing of the treated fluid can be induced by providing an appropriate twisted, elongated planar member within the passageway 145, such as a twisted ribbon of metal or plastic.

By appropriately choosing the diameter of the conduit and the material of conduit construction, the reactor 123 can be operated at a desired flow rate within the conduit so that the flow is substantially non-turbulent. With some fluids, such as with whole blood, avoidance of turbulent flow is necessary to prevent damage to the blood components.

Preferably, the helical configuration of the tubing 144 is arranged to permit the heating or cooling fluid to flow around substantially all of the exterior surface of the tubing within the tank 112. Accordingly, it is desirable to effect a relatively uniform heat transfer capability per unit tubing length throughout the configuration of helices within the tank 112. Thus, it is desired to avoid a configuration in which substantial portions of the surface of the tubing 144 are not contacted by flowing heating or cooling fluid. To this end, a substantially continuous spacer structure is provided for at least radially separating adjacent helices so as to permit circulation of the heating or cooling fluid around the tube in each helix. Preferably, as best illustrated in FIGURE 5, the tubing 144 includes a ridge member or members, such as a plurality of filaments, e.g., generally cylindrical filaments 170 and 172, extending in overlapping spiral loci around the surface of the tubing. The filaments 170 and 172 are preferably each a solid, monofilament of a suitable material, such as polyethylene, polypropylene, or nylon of a suitable diameter.

For example, it has been found that with tubing 144 having an outside diameter of 0.281 inch, a polyethylene or nylon monofilament having a diameter of 0.047 inch provides a satisfactory spacing between adjacent portions of the tubing 144 when the tubing 144 is arranged in a plurality of helices as illustrated in FIGURE 3. Preferably, the sizes of the filaments 170 and 172 and of the tubing 144, as well as the pitch of the wining of the filaments 170 and 172, are chosen such that, for a particular volumetric flow rate of the heating or cooling fluid through the tank or casing 112, the Reynolds Number that is characteristic of the heating or cooling fluid flow is well within the turbulent region. With such a design, the heating or cooling fluid passing radially outwardly from the center of the shell 114 and through the configuration of helices can have a significant effect on the temperature of the fluid flowing in the tubing 144 within a relatively small percentage of the total tubing length within the tank 112.

As an example of an effective hemoreactor constructed in accordance with the teachings of the present invention, the reactor illustrated in FIGURE 3 can have an overall length of about 13 inches (33 cm) and an outside diameter of about 4½ inches (11.4 cm). Shell 114 can be molded from DYLARK 232, a styrene-maleic anhydride copolymer, commercially available from ARCO Polymers, Inc., Philadelphia, Pa., USA, as two halves which are bonded together using methylene chloride. Other suitable materials of construction for shell 114 can be polyvinyl chloride, usually bonded together using cyclohexane, polyurethane, usually bonded together using tetrahydrofuran, and the like materials. Approximately 80—90 feet of polyvinyl chloride tubing, having an inside diameter of 0.165—0.188 inch to give a net volume of about 400 cc, and an outside diameter of 0.281 inches, is wrapped on its exterior with a pair of 0.047 inch diameter polypropylene monofilaments co-wound in overlapping spiral loci as shown in FIGURE 5 and with a pitch of about 1½ inches. The filament-wraped tubing is wound about the core 150 to form four concentric helices about the longitudinal axis of the shell 112. The diameter of the innermost helix, as measured to the centre line of the tubing 144, is about 2 inches and the diameter of the outermost helix, similarly measured to the centre line of the tubing 144, is about 4 inches. With this construction, the pressure drop through the tubing 144, as measured across the inlet and outlet ends 146 and 148 respectively, is less than 18 mm of mercury at a water flow rate of 40 ml/mm.

To further enhance heat transfer through the entire length of tubing 144, an axial baffle 179, such as a twisted strip of about 1/16-inch thick thermoplastic material or stainless steel may be disposed within the interior of the cylindrical core 151, for example, depending from core end flange 155, for deflecting the entering heating or cooling fluid radially outwardly away from the longitudinal axis of the tank 112 and

through the spaced helices of tubing 144.

Although the separating or spacer ridge members 170 and 172 are illustrated as being formed from small diameter filaments co-wound in spiral loci about the tubing 144, it is to be realized that the spacer structure may be provided integrally with the wall of the tubing 144, as with one or more outwardly projecting continuous ridges to substantially duplicate the structure of one or both of the filaments 170 and 172.

Of course, other forms of spacing means may be used, either continuous or intermittent with respect to the surface of the tube 144. As another alternative, the tubing 144 could be co-wound in its helical configuration with a strip of open-pore foam to provide a ventilated matrix spacer. Also, spacer collars, such as rings of foam, could be circumferentially disposed about each helix, at axially spaced locations within the tank to effect a radial separation of adjacent helices.

In the embodiment illustrated in FIGURE 3, the tubing 144 is closely wound in each helix so that the spiral monofilaments 170 and 172 necessarily provide axial separation of adjacent turns within the same helix as well as radial separation of adjacent concentric helices. It is to be realized that the axial separation of adjacent turns within the same helix could be maintained without the use of filaments. For example, the helices may be wound with a greater pitch to provide a relatively greater axial separation spacing between adjacent turns. The axial spacing could be maintained by co-winding the tubing 144 into the helix configuration with a coextensive spring member, such as a coiled spring, which has the desired spacing. With such relatively larger turn spacings, the outer concentric helices may tend to fill in the spacings of the inner helices. To avoid this, a generally cylindrical, foraminous screen can be placed over each completed helix. The screen could be not unlike the foraminous core member 151 illustrated in FIGURE 3, although the thickness of such a screen could be relatively thin since it need not serve to support the helix assembly, but merely radially separate adjacent helices. With the tubing co-wound with a spring member, some amount of radial and lateral separation of the tubing surfaces will be provided — even without the cylindrical screens being placed between adjacent, concentric helices.

As another example, a toroidal secondary flow blood reactor structure comprises a helical blood conduit of at least 150 radians of convolution or average turn radius of curvature not exceeding six centimeters immersed in a heat transfer bath of 35 to 45°C. During blood treatment wherein a mixture of blood and a therapy agent flow through the conduit, the mixture is maintained at about 43°C.

## Claims

1. A therapeutic composition suitable for extracorporeal treatment of whole blood and comprising a water-soluble chemotherapeutic agent and a water-soluble fluorescable tracer means, characterised in that the chemotherapeutic agent and the tracer means are dialyzable from whole blood at rates that are a function of one another, and the fluorescable tracer means is an N-substituted benzamide represented by the formula

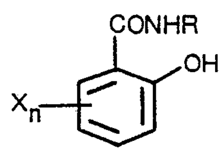

wherein R is hydrogen or methyl, X is a halogen, methoxy or ethoxy and n is an integer having a value of 0 or 1, and physiologically-tolerable alkali metal salts thereof.

2. A therapeutic composition in accordance with claim 1, characterised in that the fluorescable tracer means is salicylamide.

3. A therapeutic composition in accordance with claim 1 or claim 2, characterised in that the chemotherapeutic agent is a water-soluble cyanate.

4. A therapeutic composition suitable for extracorporeal treatment of whole blood and comprising an aqueous solution containing cyanate and salicylamide, characterised in that the cyanate is present in a concentration of 0.2 molar to 0.6 molar and the salicylamide is present in a concentration of 2.5 millimolar to 8.5 millimolar.

5. A method of treating whole blood with a dialyzable chemotherapeutic agent in an extracorporeal stream of the blood, characterised by:

(a) introducing the chemotherapeutic agent into the extracorporeal stream of blood together with a fluorescable tracer means that is dialyzable therefrom at a rate which is a function of the dialysis rate for the chemotherapeutic agent;

(b) dialyzing the extracorporeal stream so as to remove therefrom in a dialyzate the chemotherapeutic agent and the tracer means at respective rates that are a function of one another;

(c) irradiating a portion of the obtained dialyzate with fluoroescence-exciting electromagnetic radiation;

(d) passing the irradiation portion over a detector means responsive to fluoroescence emitted by the tracer means present in the said portion and generating an electrical signal indicative of the intensity of emitted fluorescence; and

(e) energizing an indicator means with the generated signal;
the fluorescable tracer means being an N-substituted benzamide represented by the formula:

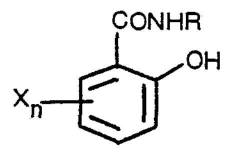

wherein R is hydrogen or methyl, X is a halogen, methoxy or ethoxy, and n is an integer having a value of 0 or 1, and physiologically-tolerable alkali metal salts thereof.

6. A method in accordance with claim 5, characterised in that the chemotherapeutic agent is a cyanate and the tracer means is salicylamide.

7. A method in accordance with claim 6, characterised in that the dialysate is maintained at a pH of about 7.4 and at a temperature of about 41°C.

8. A method in accordance with claim 5, characterised in that the dialysate portion is diluted prior to irradiation.

9. A method in accordance with claim 5, characterised in that the chemotherapeutic agent is a cyanate and is introduced into the extracorporeal stream of whole blood in an amount sufficient to provide therein a cyanate concentration of 0.01 molar to 0.03 molar, and in that the tracer means is salicylamide and is introduced into the extracorporeal stream of the whole blood in an amount sufficient to provide therein a salicylamide concentration of 0.14 millimolar to 0.4 millimolar.

10. A method in accordance with claim 5, characterised in that the chemotherapeutic agent is sodium cyanate and is introduced into the extracorporeal stream of whole blood in an amount sufficient to provide therein a cyanate concentration of about 0.02 molar, and in that the tracer means is a salicylamide and is introduced into the extracorporeal stream of whole blood in an amount sufficient to provide therein a salicylamide concentration of about 0.27 millimolar.

11. A method in accordance with claim 5, characterised in that trisodium citrate is added to the stream of whole blood as an anti-coagulant.

12. A method in accordance with claim 5, characterised by:

(a) contacting for a predetermined time period at least a portion of the dialyzed extracorporeal stream across a dialysis membrane with an aqueous secondary dialysis solution;

(b) irradiating an aliquot of the recovered aqueous secondary dialysis solution with fluorescence exciting electromagnetic radiation; and

(c) passing the irradiated aliquot over a detector means responsive to fluorescence emitted by the tracer means present in the aliquot and generating an electrical signal having a magnitude indicative of the intensity of emitted fluorescence.

13. A method in accordance with claim 12, characterised in that trisodium citrate is introduced into the extracorporeal stream in an amount sufficient to provide an anticoagulant effect.

14. A method for determining the concentration of dialyzable chemotherapeutic agent in an extracorporeal stream of whole blood containing the chemotherapeutic agent and salicylamide, characterised by:

(a) contacting at least a portion of the extracorporeal stream across a dialysis membrane with an aqueous alkaline solution devoid of salicylamide for a predetermined time period;

(b) thereafter recovering the aqueous alkaline solution;

(c) irradiating an aliquot of the recovered aqueous alkaline solution with ultraviolet radiation having a wavelength of about 318 nanometers;

(d) positioning the irradiated aliquot adjacent to a detector means generating an output signal in response to emitted radiation having a wavelength of about 410 nanometers, the signal being proportional to the intensity of the emitted radiation; and

(e) energizing an indicator means with the output signal.

**Patentansprüche**

1. Therapeutische Zusammensetzung, welche für die extrakorporale Behandlung von Gesamtblut geeignet ist und ein wasserlösliches chemotherapeutisches Mittel sowie ein wasserlösliches fluoreszierbares Tracermittel umfaßt, dadurch gekennzeichnet, daß das chemotherapeutische Mittel und das Tracermittel aus dem Gesamtblut mit Geschwindigkeiten dialysierbar sind, die eine Funktion voneinander darstellen, und das fluoreszierbare Tracermittel ein N-substituiertes Benzamid ist, welches durch die Formel

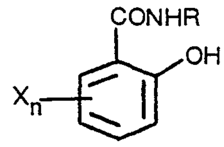

wiedergegeben wird, worin R Wasserstoff oder Methyl ist, X ein Halogen, Methoxy oder Ethoxy ist und n eine ganze Zahl ist, die den Wert 0 oder 1 besitzt, sowie physiologisch verträgliche Alkalimetallsalze desselben.

2. Therapeutische Zusammensetzung nach Anspruch 1, dadurch gekennzeichnet, daß das fluoreszierbare Tracermittel Salicylamid ist.

3. Therapeutische Zusammensetzung nach Anspruch 1 oder Anspruch 2, dadurch gekennzeichnet, daß das chemotherapeutische Mittel ein wasserlösliches Cyanat ist.

4. Therapeutische Zusammensetzung, die sich zur extrakorkorporalen Behandlung von Gesamtblut eignet und eine wässrige Lösung umfaßt, welche Cyanat und Salicylamid enthält, dadurch gekennzeichnet, daß das Cyanat in einer Konzentration von 0,2 Molar bis 0,6 Molar und das Salicylamid in einer Konzentration von 2,5 Millimolar bis 8,5 Millimilar vorliegt.

5. Verfahren zur Behandlung von Gesamtblut mit einem dialysierbaren chemotherapeutischen Mittel in einem extrakorporalen Blustrom, dadurch gekennzeichnet, daß

a) in den extrakorporalen Blutstrom das chemotherapeutische Mittel eingebracht wird zusammen mit einem fluoreszierbaren Tracermittel, welches davon mit einer Geschwindigkeit dialysierbar ist, welche eine Funktion der Dialysegeschwindigkeit des chemotherapeutischen Mittels ist;

b) der extrakorporale Strom dialysiert wird, um daraus in einem Dialysat das chemotherapeutische Mittel und das Tracermittel mit entsprechenden Geschwindigkeiten, die eine Funktion voneinander darstellen, zu entfernen;

c) ein Teil des erhaltenen Dialysats mit einer fluoreszenzanregenden elektromagnetischen Strahlung bestrahlt wird;

d) der bestrahlte Teil über eine Detektorvorrichtung bewegt wird, welche auf die Fluoreszenz anspricht, welche von dem Tracermittel, das in diesem Teil vorliegt, emitiert wird und welche ein elektrisches Signal erzeugt, welches die Intensität der emitierten Fluoreszenz anzeigt; und

e) eine Anzeigeeinrichtung mit dem erzeugten Signal erregt wird;

wobei das fluoreszierbare Tracermittel ein N-substituiertes Benzamid ist, welches durch die Formel

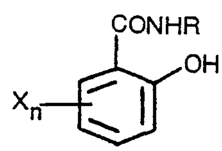

wiedergegeben wird, worin R ein Wasserstoff oder Methyl, X ein Halogen, Methoxy oder Ethoxy ist und n eine ganze Zahl ist, die den Wert 0 oder 1 besitzt, und physiologisch verträgliche Alkalisalze davon.

6. Verfahren nach Anspruch 5, dadurch gekennzeichnet, daß das chemotherapeutische Mittel ein Cyanat und das Tracermittel Salicylamid ist.

7. Verfahren nach Anspruch 6, dadurch gekennzeichnet, daß das Dialysat auf einem pH von etwa 7,4 und einer Temperatur von etwa 41°C gehalten wird.

8. Verfahren nach Anspruch 5, dadurch gekennzeichnet, daß der Dialysatanteil vor der Bestrahlung verdünnt wird.

9. Verfahren nach Anspruch 5, dadurch gekennzeichnet, daß das chemotherapeutische Mittel ein Cyanat ist und dem extrakorporalen Strom des Gesamtblutes in einer Menge zugeführt wird, die ausreicht, um darin eine Cyanatkonzentration von 0,01 Molar bis 0,03 Molar zu erhalten, und daß das Tracermittel Salicylamid ist und dem extrakorporalen Strom des Gesamtblutes in einer Menge zugeführt wird, die ausreicht, um eine Salicylamidkonzentration von 0,14 Millimolar bis 0,4 Millimolar zu erreichen.

10. Verfahren nach Anspruch 5, dadurch gekennzeichnet, daß das chemotherapeutische Mittel Natriumcyanat ist und dem extrakorporalen Strom des Gesamtblutes in einer Menge zugeführt wird, die ausreicht, um darin eine Cyanatkonzentration von etwa 0,02 Molar zu erreichen, und daß das Tracermittel Salicylamid ist und dem extrakorporalen Strom des Gesamtblutes in einer Menge zugeführt wird, die ausreicht, um darin eine Salicylamidkonzentration von etwa 0,27 Millimolar zu erreichen.

11. Verfahren nach Anspruch 5, dadurch gekennzeichnet, daß Trinatriumcitrat dem Strom des Gesamtblutes als Antikoagulanz zugesetzt wird.

12. Verfahren nach Anspruch 5, dadurch gekennzeichnet, daß

a) während eines vorgegebenen Zeitraums wenigstens ein Teil des dialysierten extrakorporalen Blutstroms durch eine Dialysemembran mit einer wässrigen sekundären Dialyselösung in Kontakt gebracht wird;

b) eine Teilmenge der gewonnen wässrigen sekundären Dialyselösung mit einer fluoreszenzanregenden elektromagentischen Strahlung bestrahlt wird; und

c) die bestrahlte Teilmenge über eine Detektorvorrichtung bewegt wird, welche auf die Fluoreszenz anspricht, die von dem Tracermittel emitiert wird, welches in der Teilmenge vorliegt, wobei ein elektrisches Signal erzeugt wird, das eine Größe aufweist, die die Intensität der emitierten Fluoreszenz anzeigt.

13. Verfahren nach Anspruch 12, dadurch gekennzeichnet, daß Trinatriumcitrat dem extrakorporalen Strom in einer Menge zugeführt wird, welche ausreicht, um einen antikoagulierenden Effekt zu erzielen.

14. Verfahren zur Bestimmung der Konzentration eines dialysierbaren chemotherapeutischen Mittels in einem extrakorporalen Strom von Gesamtblut, welches das chemotherapeutische Mittel und Salicylamid enthält, dadurch gekennzeichnet, daß

a) wenigstens ein Teil des extrakorporalen Stroms durch eine Dialysemembran mit einer wässrigen alkalischen Lösung, welche kein Salicylamid enthält, während eines vorgegebenen Zeitraums in Kontakt gebracht wird;

b) danach wie wässrige alkalische Lösung gewonnen wird;

c) eine Teilmenge der gewonnenen wässrigen alkalischen Lösung mit Ultraviolettstrahlung, welche eine Wellenlänge von etwa 318 nm aufweist, bestrahlt wird;

d) die bestrahlte Teilmenge an einer Detektorvorrichtung angeordnet wird, welche ein Ausgangssignal in Abhängigkeit von der emitierten Strahlung mit einer Wellenlänge von etwa 410 nm erzeugt, wobei das Signal der Intensität der emitierten Strahlung proportional ist; und

e) eine Anzeigevorrichtung mit dem Ausgangssignal erregt wird.

**Revendications**

1. Composition thérapeutique contenant au traitement extracorporel du sang entier et comprenant un agent chiomiothérapeutique hydrosoluble et des moyens marqueurs hydrosolubles fluorescents, caractérisée en ce que l'agent chimiothérapeutique et les moyens marqueurs sont dialysables à partir du sang entier à des débits qui dépendent l'un de l'autre, et les moyens marqueurs fluorescents sont un benzamide à substitution N, représenté par la formule

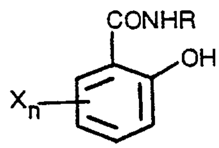

dans laquelle R est de l'hydrogène, ou un groupe méthyle, X est un halogène, un groupe méthoxy ou éthoxy, et n est un entier ayant une valeur de 0 ou 1, et leurs sels de métaux alcalins, physiologiquement tolérables.

2. Composition thérapeutique selon la revendication 1, caractérisée en ce que les moyens marqueurs fluorescents sont du salicylamide.

3. Composition thérapeutique selon la revendication 1 ou la revendication 2, caractérisée en ce que l'agent chimiothérapeutique est un cyanate hydrosoluble.

4. Composition thérapeutique convenant au traitement extracorporel du sang entier et comprenant une solution aqueuse contenant un cyanate et un salicylamide, caractérisée en ce que le cyanate est présent à une concentration de 0,2 mole à 0,6 mole et le salicylamide est présent à une concentration de 2,5 mmoles à 8,5 mmoles.

5. Procédé de traitement du sang entier avec un agent chimiothérapeutique dialysable dans un courant extracorporel de sange, caractérisé en ce qu'il consiste:

(a) à introduite l'agent chimiothérapeutique dans le courant extracorporel de sang, en même temps qu'un moyen traceur fluorescent qui peut en être dialyse à une vitesse qui est une fonction de la vitesse de dialyse de l'agent chimiothérapeutique;

(b) à dialyser le courant extracorporel afin d'en éliminer, sous forme d'un catalyst, l'agent chimiothérapeutique et le moyen marqueur à des vitesses respectives qui sont fonction l'une de l'autre;

(c) à irradier une partie du dialysat obtenu avec un rayonnement électromagnétique excitant la fluorescence;

(d) à faire passer la partie irradiée sur des moyens détecteurs sensibles à la fluorescence émise par le moyen marqueur présent dans ladite partie et à générer un signal électrique représentatif de l'intensité de la fluorescence émise; et

(e) à exciter un moyen indicateur avec le signal généré;

le moyen marqueur fluorescent étant un benzamide à substitution N représenté par la formule:

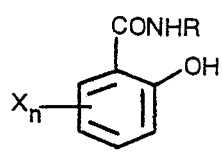

dans laquelle R est de l'hydrogène ou un groupe méthyle, X est un halogène, un groupe méthoxy ou

éthoxy, et n est un entier ayant une valeur de 0 ou 1, et leurs sels de métaux alcalins, physiologiquement tolérables.

6. Procédé selon la revendication 5, caractérisé en ce que l'agent chimiothérapeutique est un cyanate et le moyen marqueur est un salicylamide.

7. Procédé selon la revendication 6, caractérisé en ce que le dialysat est maintenu à un pH d'environ 7,4 et une température d'environ 41°C.

8. Procédé selon la revendication 5, caractérisé en ce que la partie formée du dialysat est diluée avant l'irradiation.

9. Procédé selon la revendication 5, caractérisé en ce que l'agent chimiothérapeutique est un cyanate et est introduit dans le courant extracorporel de sang entier en quantité suffisante pour y établir une concentration de cyanate de 0,01 mole à 0,03 mole, et en ce que le moyen marqueur est du salicylamide et est introduit dans le courant extracorporel de sang entier en quantité suffisante pour y établir une concentration de salicylamide de 0,14 mmole à 0,4 mmole.

10. Procédé selon la revendication 5, caractérisé en ce que l'agent chimiothérapeutique est du cyanate de sodium et est introduit dans le courant extracorporel de sang entier en quantité suffisante pour y établir une concentration de cyanate d'environ 0,02 mole, et en ce que le moyen marqueur est du salicylamide et est introduit dans le courant extracorporel de sang entier en quantité suffisante pour y établie une concentration de salicylamide d'environ 0,27 mmole.

11. Procédé selon la revendication 5, caractérisé en ce que du citrate trisodique est ajouté au courant de sang entier en tant qu'anticoagulant.

12. Procédé selon la revendication 5, caractérisé en ce qu'il consiste:

(a) à mettre en contact, pendant une période de temps prédéterminée, au moins une partie du courant extracorporel dialysé à travers une membrane de dialyse, avec une solution aqueuse de dialyse secondaire;

(b) à irradier une portion aliquote de la solution aqueuse de dialyse secondaire récupérée avec un rayonnement électromagnétique excitant la fluorescence; et

(c) à faire passer la portion aliquote irradiée sur un moyen de détection sensible à la fluorescence émise par le moyen marqueur présent dans la portion aliquote et à générer un signal électrique ayant une amplitude représentative de l'intensite de la fluorescence émise.

13. Procédé selon la revendication 12, caractérisé en ce que du citrate trisodique est introduit dans le courant extracorporel en quantité suffisante pour produire un effet anticoagulant.

14. Procédé pour déterminer la concentration d'un agent chimiothérapeutique dialysable dans un courant extracorporel de sang entier contenant l'agent chimiothérapeutique et du salicylamide, caractérisé en ce qu'il consiste:

(a) à mettre en contact au moins une partie du courant extracorporel, à travers une membrane de dialyse, avec une solution alcaline aqueuse dépourvue de salicylamide pendant une période de temps prédéterminée.

(b) à récupérer ensuite la solution alcaline aqueuse;

(c) à irradier une portion aliquote de la solution alcaline aqueuse récupérée avec un rayonnement ultraviolet ayant une longueur d'onde d'environ 318 nanomètres;

(d) à positionner la portion aliquote irradiée à proximité à proximité immédiate d'un moyen de détection générant un signal de sortie en réponse au rayonnement émis ayant une longueur d'onde d'environ 410 nanomètres, le signal étant proportionnel à l'intensité du rayonnement émis; et

(e) à exciter un moyen indicateur avec le signal de sortie.

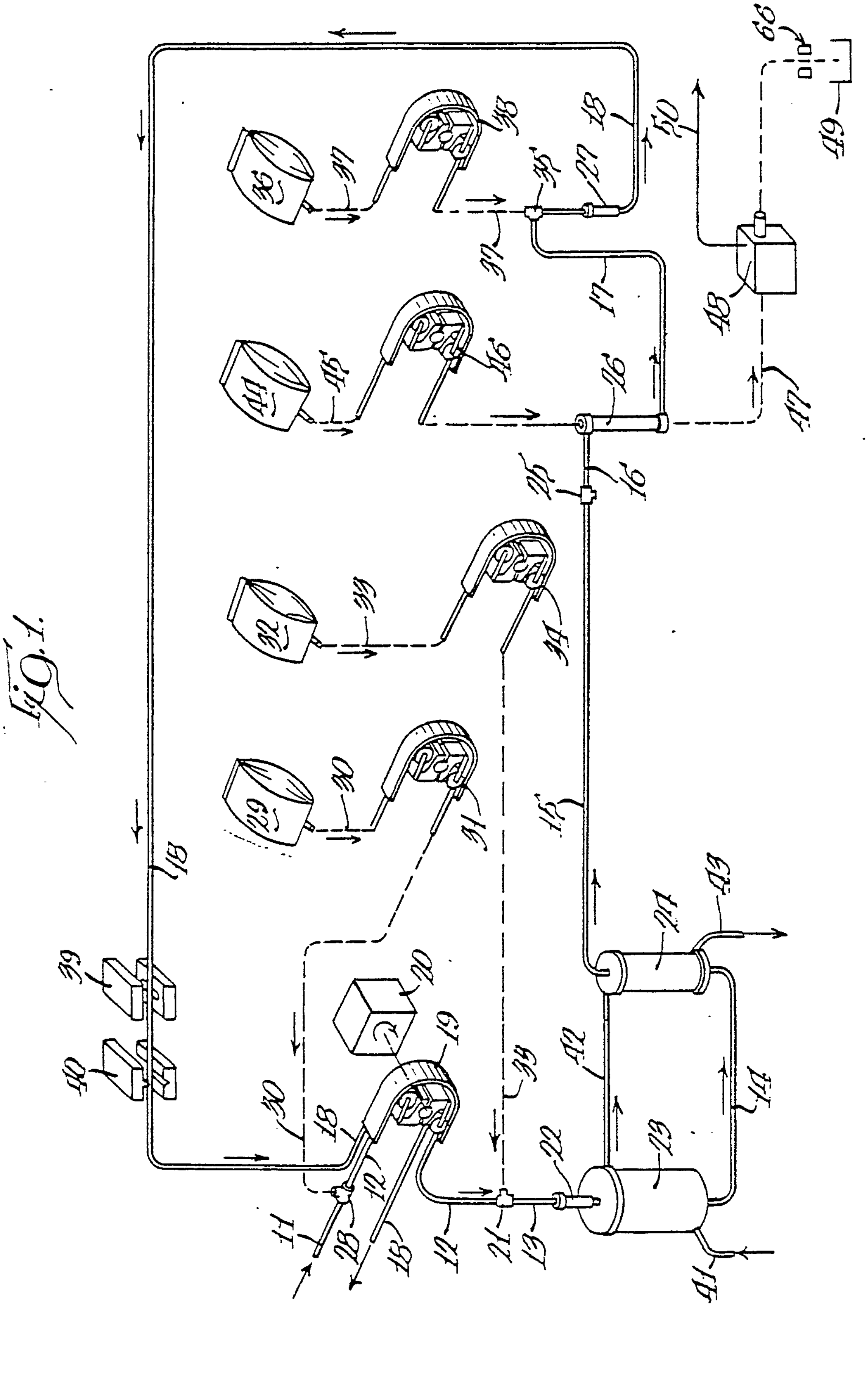

Fig. 1.

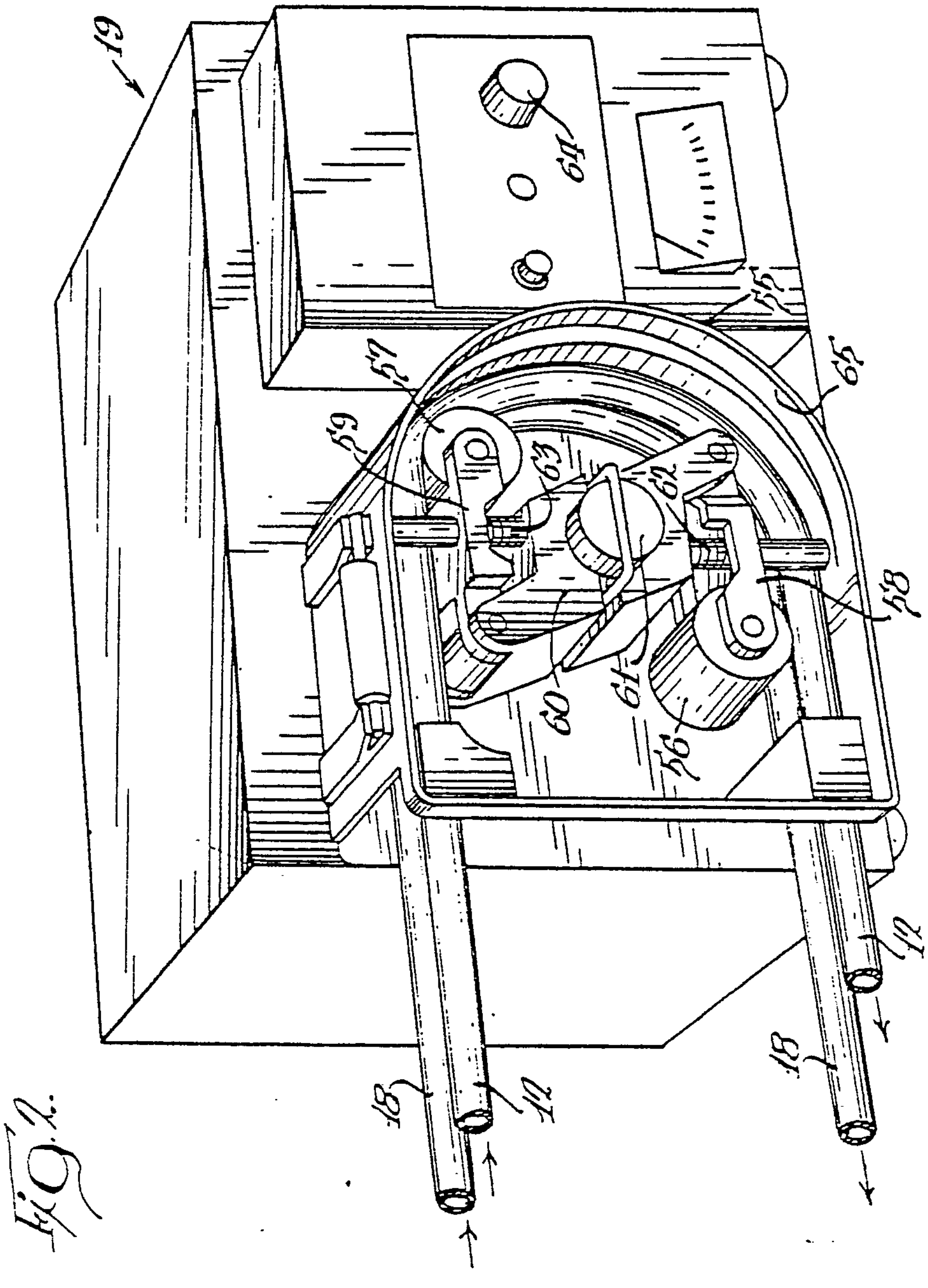


Fig. 2.

Fig. 3.

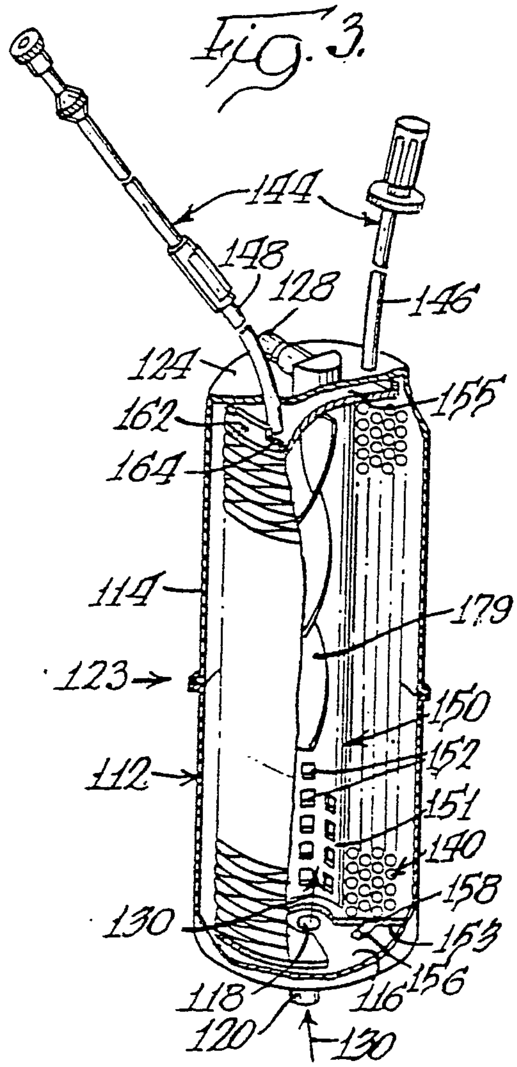


Fig. 4.

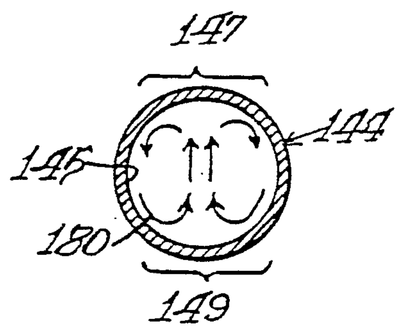


Fig. 5.